# EUROPEAN PATENT APPLICATION

(11) **EP 4 431 605 A1**
(43) Date of publication of application: **18.09.2024**
(21) Application number: 22892854.5
(22) Date of filing: 10.11.2022
(51) Int. Cl.: C12N 15/12, A61K 35/17, A61K 35/545, A61P 35/00, C12N 5/10, C12N 15/24, C12N 15/62, C12N 15/63

(54) **GENE-MODIFIED PLURIPOTENT STEM CELL, IMMUNOCOMPETENT CELL DERIVED THEREFROM, METHOD FOR PRODUCING SAID CELLS, AND USE THEREOF**

(30) Priority: 11.11.2021 JP 2021184197
(71) Applicant: Healios K.K., Tokyo 100-0006 (JP)
(72) Inventor: TAMURA, Kouichi, Tokyo 100-0006 (JP); KIMURA, Hironobu, Tokyo 100-0006 (JP); HOSOYA, Tomonori, Tokyo 100-0006 (JP); YAMADA, Masashi, Tokyo 100-0006 (JP)
(74) Representative: Weickmann & Weickmann PartmbB
(86) International application number: PCT/JP2022/041884
(87) International publication number: WO 2023/085356

(57) **Abstract**

The present invention provides a pluripotent stem cell expressing the following (a) and (b):
(a) an exogenous gene encoding CC chemokine receptor 2 type B(CCR2B)
(b) an exogenous gene encoding CC chemokine ligand 19 (CCL19),
or an NK cell derived from the pluripotent stem cell or a progenitor cell thereof.

## Description

### [Technical Field]

The present invention relates to a genetically modified pluripotent stem cell, an immunocompetent cell derived from the pluripotent stem cell, method of producing same and use thereof and the like. More particularly, the present invention relates to a genetically modified pluripotent stem cell with enhanced function for cellular immunotherapy, a natural killer (NK) cell or progenitor cell thereof derived from the pluripotent stem cell, a method of producing same comprising introducing exogeneous genes for enhancing the function into a pluripotent stem cell, and use thereof as a cell medicine and the like.

### [Background Art]

Cells involved in cancer immunity include lymphocytes such as T cell, NK cell, natural killer T (NKT) cell and the like. Research and development of cell therapy using these immune cells has a history not less than half a century. In recent years, developments of iPS cell and methods for inducing differentiation therefrom, as well as technology developments such as gene modification methods of iPS cell have made it possible to genetically modify an iPS cell and introduce differentiation into an immune cell of interest, thereby producing an immune cell on which high function is conferred. As such various therapeutic strategies using an immune cell could have been constructed.

As cell pharmaceuticals of genetically modified immune cell mentioned above, those using CAR-T cell targeting CD19 (Kymriah and Yescarta) have been approved to date. In the meantime, NK cell does not require presensitization against cancer cells, and can respond without restriction of antigen recognition. NK cell directly recognizes and kills a target cell. Furthermore, it has a function that activates T cell and macrophage and the like via production of various cytokines to improve immune function. Also, unlike T cell, NK cell less likely causes problems such as GvHD even if using a donor cell with incomplete HLA type-matching. Furthermore, it less likely causes cytokine release syndrome (CRS), which is a side effect due to T cell. As such, in view of good results of T cell therapy in recent years, expectations for immune cell therapy using NK cell, which is also an antitumor effector cell, have also been growing.

In fact, there are various clinical trials aimed at cell pharmaceuticals using modified NK cells (Non Patent Literature 1). For example, Fate Therapeutics has been developing a modified NK cell derived from an iPS cell, in which CD38 is knocked-out (KO) and an IL-15/IL-15 receptor fused protein is expressed, targeting solid tumor (e.g., Patent Literature 1). Takeda Pharmaceutical Company Limited has been developing a CD19-CAR NK cell expressing IL-15, together with The University of Texas MD Anderson Cancer Center (e.g., Non Patent Literature 2). However, nothing has been approved by concerned authorities.

As function-enhancing factors used for NK cell, IL-15, which is introduced into the above-mentioned developed products by Fate Therapeutics and Takeda Pharmaceutical Company Limited, CD16 and the like are known. It is known that IL-15 proliferates and activates NK cell per se, and also activates the surrounding T cells (Non Patent Literatures 3-5). Furthermore, CD16 is known to contribute to the antibody-dependent cellular cytotoxicity (ADCC) action mediated by NK cells by being expressed on the surface of NK cells (Non Patent Literature 6). Therefore, attempts to express these factors, whose functions in NK cells are already well known, in NK cells and further enhance their functions have already been adopted in various modified NK cells, including products developed by Fate Therapeutics and Takeda Pharmaceutical Co., Ltd. mentioned above.

On the other hand, there are attempts to increase the ability to attack cancer cells by expressing various chemokines and chemokine receptors in immune cells and increasing the migration ability thereof. For example, Patent Literature 2 discloses cancer immunotherapy using T cells expressing various chemokines and chemokine receptors such as CCR2. Also, Patent Literature 3 discloses that the antitumor activity of T cells is enhanced and the proliferation property is improved by expressing CCL19 together with various interleukins. Furthermore, Patent Literature 4 discloses CAR immune cells that express immune activation factors, and describes that NK cells are also targeted as CAR immune cells. In addition, IL-15 and CCR2B are listed as immune activation factors.

However, these disclosures only relate to methods for expressing those factors in immune cells such as T cells and enhancing the functions thereof, and do not utilize genetically modified iPS cells. Furthermore, none of the Literatures include description suggesting introduction of a combination of CCR2B and CCL19 into NK cells.

### [Citation List]

### [Patent Literature]

[Patent Literature 1]
   WO 2019/126748
[Patent Literature 2]
   WO 2018/152572
[Patent Literature 3]
   WO 2020/045610
[Patent Literature 4]
   WO 2017/133633

### [Non Patent Literature]

[Non Patent Literature 1]
   Nat Rev Drug Discov. 2020 Mar; 19(3):200-218
[Non Patent Literature 2]
   N Engl J Med. 2020 Feb 6; 382(6): 545-553
[Non Patent Literature 3]
   J Exp Med. 1994 Oct 1; 180(4): 1395-1403
[Non Patent Literature 4]
   Cancer Immunnol Immunother. 2012 Sep; 61(9): 1451-1461
[Non Patent Literature 5]
   Nat Rev Immunnol. 2003 Apr; 3(4): 269-279
[Non Patent Literature 6]
   Front Immunnol. 2015 Jul 27; 6: 368

### [Summary of Invention]

### [Technical Problem]

Genetically modified immune cells that become a further therapeutic option are demanded. Therefore, the present invention aims to provide genetically modified immunocompetent cells having high functions, particularly, genetically modified NK cells derived from pluripotent stem cells such as iPS cells.

### [Solution to Problem]

In order to achieve the above-mentioned purposes, the present inventors came up with the idea of highly expressing chemokines and chemokine receptors in NK cells. Therefore, from among the many chemokines and chemokine receptors, CCR2B was selected as a chemokine receptor and CCL19 was selected as a chemokine and these were exogenously introduced into iPS cells by genetic modification. The iPS cells were induced to differentiate into NK cells and the functions thereof were verified. As a result, it was found that the modified NK cells exhibited higher functions than expected. It is known that, in immune cells, cytokines including chemokines may have dual properties regarding cancer immunity. For example, CCL2 can promote the recruitment of immunosuppressive cells and suppress functions of T cells (e.g., Cancer Lett 2007 Jul 8; 252(1):86-92.). That is, even if a plurality of chemokines/chemokine receptors are combined, it is completely unknown whether or not the expected bonus effect can be obtained, and the findings by the present inventors are surprising.

Based on these findings, the present inventors have conducted further studies and completed the present invention.

Accordingly, the present invention provides the following.

### Item [1]

A pluripotent stem cell expressing the following (a) and (b):
(a) an exogenous gene encoding CC chemokine receptor 2 type B (CCR2B)
(b) an exogenous gene encoding CC chemokine ligand 19 (CCL19),
or an NK cell derived from the pluripotent stem cell, or a progenitor cell thereof.

### Item [2]

The cell according to item [1], further expressing the following (c) and/or (d):
(c) an exogenous gene encoding interleukin 15 (IL-15)
(d) an exogenous gene encoding CD16.

### Item [3]

The cell according to item [1] or [2], further expressing the following (e) :
(e) an exogenous gene encoding NKG2D or NKG2D-CAR.

### Item [4]

The cell according to item [3], wherein (e) is an exogenous gene encoding NKG2D-CAR.

### Item [5]

The cell according to item [3], wherein (e) is an exogenous gene encoding NKG2D, further co-expressing:
(f) an exogenous gene encoding DAP10.

### Item [6]

The cell according to any one of items [2] to [5], expressing an exogenous gene encoding IL-15, wherein the IL-15 is a secretory IL-15.

### Item [7]

The cell according to any one of items [2] to [5], expressing an exogenous gene encoding IL-15, and co-expressing an exogenous gene encoding IL-15 receptor α (IL-15Rα).

### Item [8]

The cell according to any one of items [2] to [7], expressing an exogenous gene encoding CD16, wherein the CD16 is CD16 having a high affinity mutation or a non-cleavage mutation.

### Item [9]

The cell according to item [8], wherein the CD16 has a mutation selected from F176V (F158V) and S197P.

### Item [10]

A medicament comprising the cell according to any one of items [1] to [9].

### Item [11]

The medicament according to item [10], which is a therapeutic drug for cancer.

### Item [12]

A method of producing a genetically modified cell with enhanced homing function for cancer tissue and immunocompetent cell-recruitment function, comprising introducing the following (a) and (b) :
(a) an exogenous gene encoding CCR2,
(b) an exogenous gene encoding CCL19
into a pluripotent stem cell.

### Item [13]

The method according to item [12], further comprising inducing differentiation of the pluripotent stem cell, into which the exogeneous genes have been introduced, into NK cell or a progenitor cell thereof.

### Item [14]

A construct for producing a genetically modified cell, comprising the following (a) and (b):
(a) an exogenous gene encoding CCR2B
(b) an exogenous gene encoding CCL19.

### Item [15]

The construct according to item [14], wherein the exogeneous genes are contained in separate constructs.

### Item [16]

An expression cassette comprising a regulatory region for gene expression and the construct according to item [14] or [15] under the control of the regulatory region.

### Item [17]

A vector comprising the expression cassette according to item [16] .

### Item [18]

The vector according to item [17], having a structure in which the expression cassette is flanked by a pair of transposon inverted repeat sequences.

### Item [19]

A kit for producing a genetically modified cell that expresses the following (a) and (b):
(a) an exogenous gene encoding CCR2B
(b) an exogenous gene encoding CCL19,
comprising the vector according to item [18] and a transposase-expressing vector.

### Item [20]

The kit according to item [19], wherein the transposase is PiggyBac transposase.

### Item [21]

A method for treating cancer comprising administering the cell according to any one of items [1] to [9] to a cancer patient.

### Item [22]

The cell according to any one of items [1] to [9] for use in a method for treating cancer.

### Item [23]

Use of the cell according to any one of items [1] to [9] in the manufacture of a medicament for treating cancer.

### [Advantageous Effects of Invention]

The modified NK cells or progenitor cells thereof of the present invention are derived from pluripotent stem cells into which genes for particularly enhancing the functions aiming at cancer immunity have been introduced. The cells have, due to the expression of CCR2B and CCL19, a homing function to cancer tissues which is superior to that of conventional cells and a function to recruit immunocompetent cells such as T cells, and have high antitumor activity. In addition, antibody-dependent cellular cytotoxicity is enhanced by the high expression of CD16, or sustainability and proliferation property are improved by the expression of IL-15, and further, IL-15 receptor α. In addition, high expression of NKG2D(-CAR), and further, DAP10 affords enhanced activation signals due to NKG2D ligand stimulation.

Therefore, by using the modified NK cells or progenitor cells thereof of the present invention, the therapeutic effect of immunotherapy can be improved. Furthermore, the modified NK cells or progenitor cells thereof of the present invention may be involved in the activation of not only the NK cells themselves but also surrounding immune cells such as T cells. This suggests the possibility of being useful for the treatment of diseases for which the effects are limited by existing treatment methods.

In addition, since the modified NK cells or progenitor cells thereof of the present invention can be obtained by inducing differentiation of genetically modified pluripotent stem cells, large amounts of modified NK cells or progenitor cells thereof can be produced more easily than the cells obtained by genetically modifying NK cells.

According to the combination of the predetermined factors of the present invention, various modified immune cells with high antitumor activity can be obtained even when genes are introduced and expressed in immune cells in general, not only NK cells as described above, but also T cells, monocytes/macrophages, dendritic cells, and the like.

### [Brief Description of Drawings]

[Fig. 1]

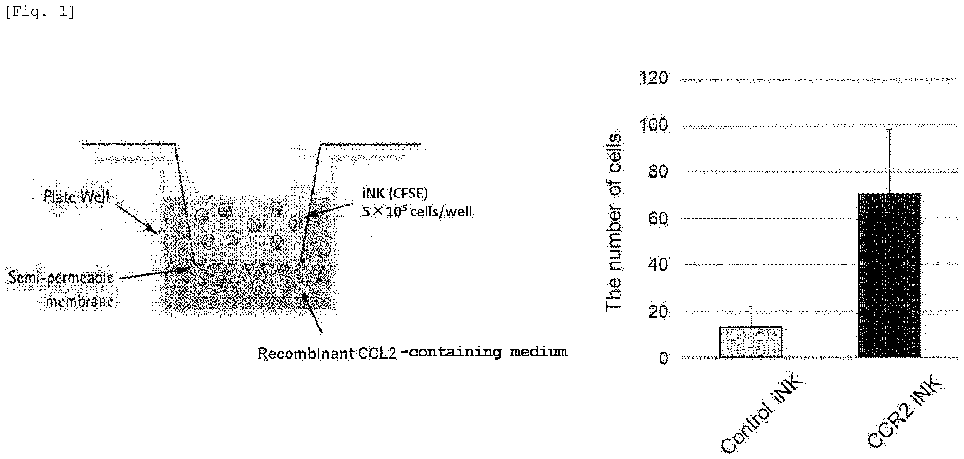

   Fig. 1 is a diagram showing that CCR2B-expressing iNK cell has a high migration activity against CCL2.
[Fig. 2]

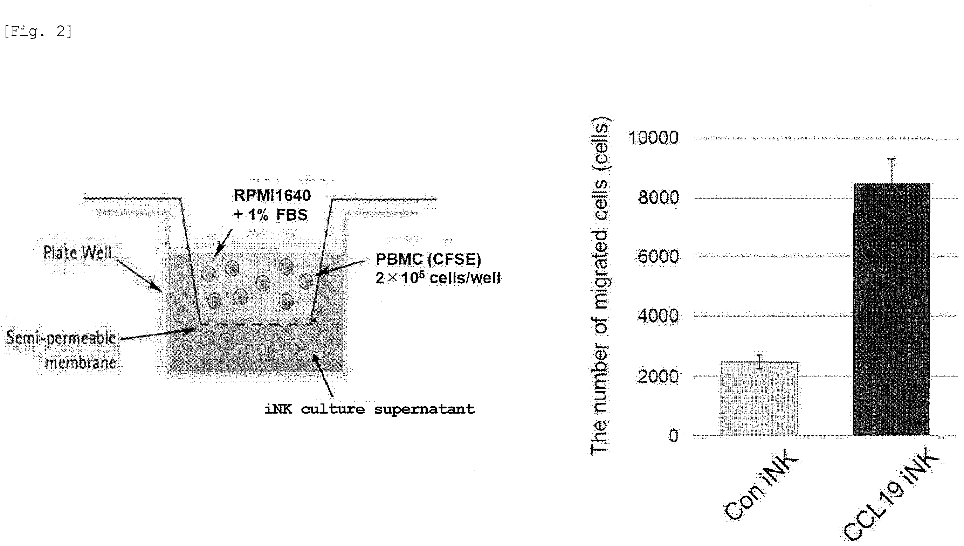

   Fig. 2 is a diagram showing that CCL19-expressing iNK cell has the ability to promote migration activity against PBMC.
[Fig. 3]

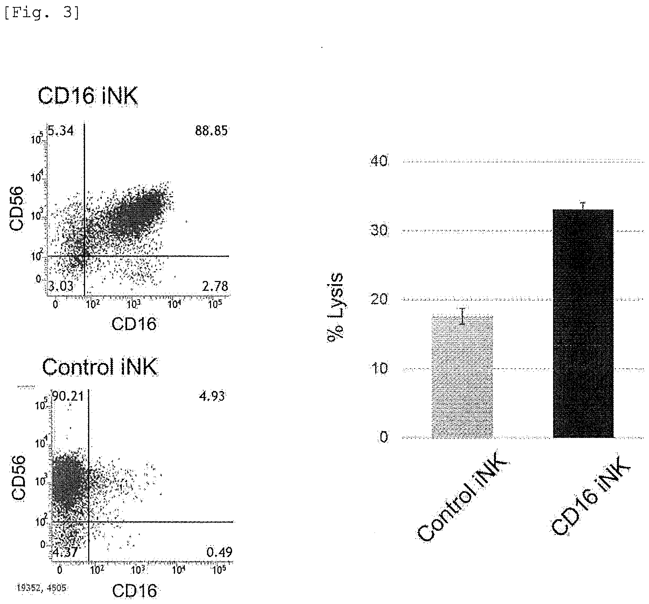

   Fig. 3 is a diagram showing that CD16-expressing iNK cell has an enhanced cytotoxic activity in the presence of an anti-EGFR antibody (Cetuximab).
[Fig. 4]

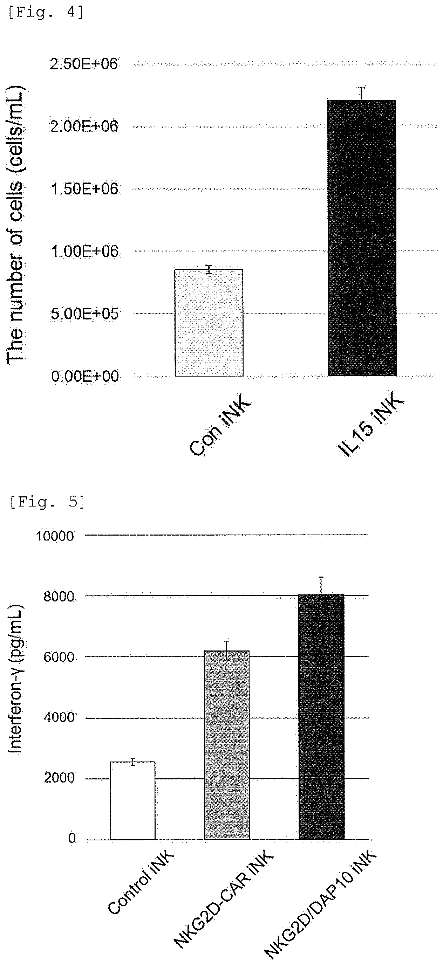

   Fig. 4 is a diagram showing that IL-15-expressing iNK cell does not reduce its cell number even when cultured in the absence of IL-15.
[Fig. 5]
   Fig. 5 is a diagram showing that IFN-γ-secretion activity of NKG2D-expressing iNK cell is enhanced by stimulation of co-culture with A549 cell.
[Fig. 6]

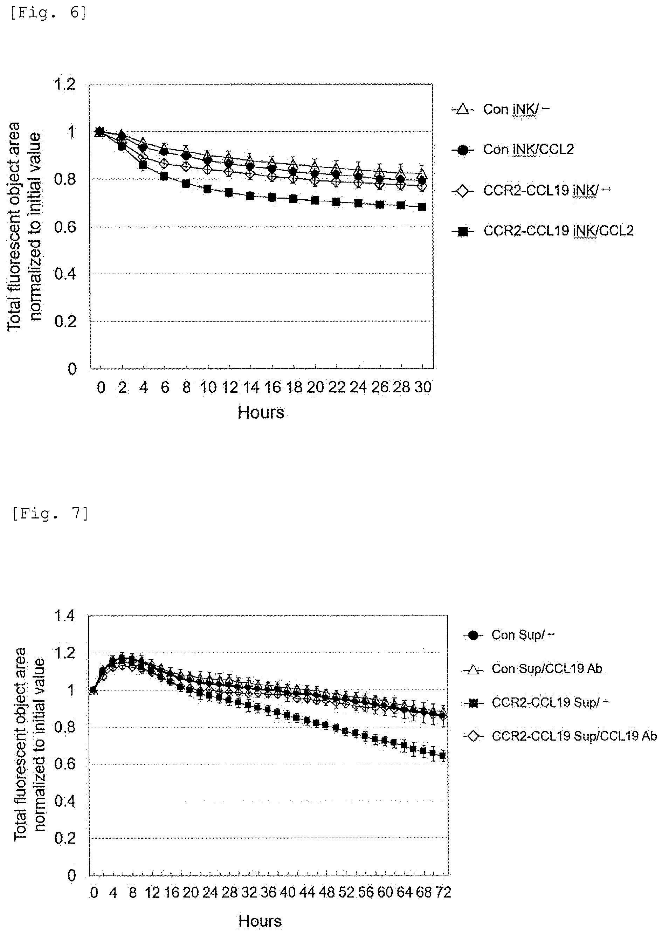

   Fig. 6 is a diagram showing that CCR2B/CCL19-co-expressing iNK cell has high migration ability against CCL2.
[Fig. 7]
   Fig. 7 is a diagram showing that CCR2B/CCL19-co-expressing iNK cell has a high attracting activity against DC.
[Fig. 8]

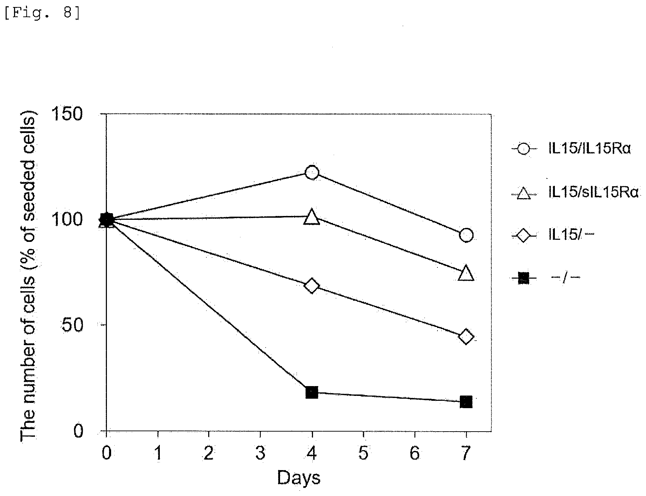

   Fig. 8 is a diagram showing that IL2sp/IL-15 expression improves proliferation and survival of iNK cell, and co-expression of full-length or soluble IL-15Rα further increases the effect.

### [Description of Embodiments]

### (I) Genetically modified pluripotent stem cell of the present invention

The present invention provides a genetically modified pluripotent stem cell suitable for differentiation induction into genetically modified NK cell or a progenitor cell thereof with enhanced functions as an immunocompetent cell (hereinafter also to be referred to as "the modified pluripotent stem cell of the present invention") . The pluripotent stem cell is a pluripotent stem cell expressing the following (a) and (b):
(a) an exogenous gene encoding CC chemokine receptor 2 type B (CCR2B)
(b) an exogenous gene encoding CC chemokine ligand 19 (CCL19).

Since target cells such as cancer cells express CC chemokine ligand 2 (CCL2), when its receptor, CCR2B is introduced into and expressed in a pluripotent stem cell, it is possible to home NK cell differentiation-induced from the pluripotent stem cell to a target tissue such as cancer tissue.

On the other hand, CCL19 is a ligand for CC chemokine receptor 7 (CCR7). Since CCR7 expresses in T cell or dendritic cell, when CCL19 is introduced into and expressed in a pluripotent stem cell, NK cell differentiation-induced from the pluripotent stem cell can stimulate migration of immunocompetent cells such as T cell or dendritic cell surrounding the cell to improve the effects of NK cell.

The "pluripotent stem cell" as used herein means having pluripotency, ES cell, iPS cell and the like are exemplified. Preferably, it is iPS cell. In the present specification, NK cell differentiation-induced from an iPS cell is called as "iNK cell".

An ES cell can be produced by a method known per se. The methods for producing ES cells include, but are not limited to, for example, a method of culturing the inner cell mass at the human blastocyst stage (e.g., Manipulating the Mouse Embryo: A Laboratory Manual, Second Edition, Cold Spring Harbor Laboratory Press (1994)), a method of culturing an early embryo produced by somatic cell nuclear transplantation (Wilmut et al., Nature, 385, 810 (1997); Cibelli et al., Science, 280, 1256 (1998); valley, protein nucleic acid enzyme, 44, 892 (1999); Baguisi et al., Nature Biotechnology, 17, 456 (1999); Wakayama et al., Nature, 394, 369 (1998); Wakayama et al., Nature Genetics, 22, 127 (1999); Wakayama et al., Proc. Natl. Acad. Sci. USA, 96, 14984 (1999); RideoutIII et al., Nature Genetics, 24, 109 (2000)), and the like. In addition, ES cells can be obtained from specified institutions, and commercially available products can also be purchased. For example, human ES cell lines H1, H7, H9, H13 and H14 are available from the WiCell Research Institute in the United States; HES1 - 6 are available from the ES Cell International in Australia, SA002, SA181, and SA611 are available from Cellartis AB in Sweden, HUES1 - 17 are available from HUES Cell Facility in the United States, KhES-1 - KhES-5 are available from The Institute for Frontier Life and Medical Sciences, Kyoto University, and SEES1 - SEES7 are available from the National Center for Child Health and Development. When ES cells are produced by somatic cell nuclear transplantation, the type of somatic cells and the source from which somatic cells are collected are in accordance with the following case of iPS cell production.

iPS cell is an artificial stem cell derived from somatic cells and having properties almost equivalent to those of ES cells, for example, differentiation pluripotency and proliferation potency by self-renewal, and it can be produced by introducing specific reprogramming factors in the form of nucleic acids (DNA or RNA) or proteins into somatic cells (Takahashi, K. and S. Yamanaka (2006) Cell, 126: 663-676; Takahashi, K. et al. (2007) Cell, 131: 861-872; Yu, J. et al. (2007) Science, 318: 1917-1920; Nakagawa, M. et al. (2008) Nat. Biotechnol. 26: 101-106; WO2007/069666).

The term "somatic cell" used in the present specification means any human cell excluding germ line cells and totipotent cells such as ovum, oocyte, ES cell, and the like. Somatic cells non-limitatively include any of fetal somatic cells, neonatal somatic cells, and mature healthy or diseased somatic cells, as well as any primarily-cultured cells, subcultured cells, and established lines of cells. Specific examples of the somatic cell include (1) tissue stem cells (somatic stem cells) such as nerve stem cells, hematopoietic stem cells, mesenchymal stem cells, and dental pulp stem cells, (2) tissue progenitor cell, (3) differentiated cells such as lymphocyte, epithelial cell, endothelial cell, muscle cells, fibroblast (dermal cell etc.), hair cell, hepatocyte, gastric mucosal cell, enterocyte, splenocyte, pancreatic cell (pancreatic exocrine cell etc.), brain cell, lung cell, kidney cell, adipocyte, and the like, and the like.

The reprogramming factors may be composed of genes specifically expressed in ES cells, gene products thereof, or non-coding RNA, or gene that plays an important role in maintaining an undifferentiated state of ES cells, gene products thereof, or non-coding RNA, or low-molecular-weight compounds. Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2, Tbx3, Glis1 and so on. These reprogramming factors may be used alone or in combination. Examples of the combination of the reprogramming factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu, D. et al. (2008) Nat. Biotechnol., 26: 795-797, Shi, Y. et al. (2008) Cell Stem cell, 2: 525-528, Eminli, S. et al. (2008) Stem Cells, 26: 2467-2474, Huangfu, D. et al. (2008) Nat. Biotechnol., 26: 1269-1275, Shi, Y. et al. (2008) Cell Stem Cell, 3: 568-574, Zhao, Y. et al. (2008) Cellstem Cell, 3: 475-479, Marson, A. (2008) Cell Stem Cell, 3: 132-135, Feng, B. et al. (2009) Nat. Cell Biol., 11: 197-203, Judson, R.L. et al. (2009) Nat. Biotechnol., 27: 459-461, Lyssiotis, C.A. et al. (2009) Proc. Natl. Acad. Sci. USA, 106: 8912-8917, Kim, J.B. et al. (2009) Nature, 461: 649-643, Ichida, J.K. et al. (2009) Cell Stem Cell, 5: 491-503, Heng, J.C. et al. (2010) Cell Stem Cell, 6: 167-74, Han, J. et al. (2010) Nature, 463: 1096-100, Mali, P. et al. (2010) Stem Cells, 28: 713-720, and Maekawa, M. et al. (2011) Nature, 474: 225-9.

Selection of iPS cell colony can be performed by a method using drug resistance and reporter activity as indices (Cell, 126, 663-676 (2006), Nature, 448, 313-317 (2007)) and a method by visual observation of morphology (Cell, 131, 861-872 (2007)). iPS cells can be confirmed using the expression of various ES cell-specific genes and teratoma formation as indices.

As induced pluripotent stem cell lines, any of various human iPSC lines established by given organizations such as the US National Institute of Health (NIH), RIKEN, Kyoto University and the like can be used. For example, HiPS-RIKEN-1A strain, HiPS-RIKEN-2A strain, HiPS-RIKEN-12A strain, Nips-B2 strain, and the like of RIKEN, and 253G1 strain, 253G4 strain, 1201C1 strain, 1205D1 strain, 1210B2 strain, 1383D2 strain, 1383D6 strain, 201B7 strain, 409B2 strain, 454E2 strain, 606A1 strain, 610B1 strain, 648A1 strain, 1231A31 strain, FfI-01s04 strain, and the like of Kyoto University can be mentioned.

In the present specification, the introduction of an exogenous gene means that the exogenous gene can be expressed in the target cell by introducing the exogenous gene into the target site of the genome. As a specific means of introducing an exogenous gene, the DNA of the exogenous gene is isolated according to a conventional method, and a DNA fragment of the exogenous gene is inserted into, for example, the target site of the target cell, thereby consequently constructing a DNA strand having a DNA sequence such that the exogenous gene is expressed in the target cell (hereinafter to be abbreviated as targeting vector for gene transfer), and a method of integrating the DNA strand into the target site of the target cell by homologous recombination may be preferably used. According to the homologous recombination method, the gene insertion site is fixed. Therefore, if there is no random integration, it is expected that the difference in the expression level between clones will be small and an influence on other genes will be less.

The homologous recombinant cell can be obtained, for example, by introducing the above-mentioned targeting vector into target cells.

For example, when the targeting vector for gene transfer is designed to express the exogenous gene in the target cells by inserting DNA fragment of exogenous gene (which can be cloned by conventional methods based on the sequence information of human CCR2B gene cDNA (see, e.g., Refseq NM_001123396) and human CCL19 gene cDNA (see, e.g., Refseq NM_006274)) into the target site, the vector can have, for example, the following constitution.

First, since the DNA fragment of the exogenous gene is inserted into the target site by homologous recombination, the targeting vector for gene transfer needs to contain sequences homologous to the target site (5'-arm and 3'-arm), at the 5'-upstream and the 3'-downstream of the DNA fragment of the exogenous gene.

For selection of target cells in which the targeting vector for gene transfer has been integrated into the chromosome, targeting vector for gene transfer preferably contains drug resistance gene or reporter gene in addition to the exogenous gene to be inserted. Examples of the drug resistance gene include, but are not limited to, neomycin phosphotransferase II (nptII) gene, hygromycin B phosphotransferase (hph) gene, and the like, and Examples of the reporter gene include β-galactosidase (lacZ) gene, chloramphenicol acetyltransferase (cat) gene, and the like.

The drug resistance or reporter gene is preferably under the control of any regulatory region for gene expression that can function in the target cell. Examples of the regulatory region for gene expression include, but are not limited to, virus promoters such as SV40-derived early promoter, cytomegalovirus (CMV) long terminal repeat (LTR), Rous sarcoma virus (RSV) LTR, mouse leukemia virus (MoMuLV) LTR, adenovirus (AdV)-derived early promoter, and the like, and β-actin gene promoter, PGK gene promoter, transferrin gene promoter, and the like.

In addition, the targeting vector for gene transfer preferably has poly A signal at the downstream of the gene. For example, terminator sequences derived from viral genes or derived from various mammalian or avian genes can be used. Preferably, an SV40-derived terminator sequence or the like is used.

Generally, gene recombination in cells is largely non-homologous, and the introduced DNA is randomly inserted into any site in the chromosome. Therefore, it is not possible to efficiently select only the clones with homologous recombination at the target site by selection (positive selection) such as detection of drug resistance or reporter gene expression, and confirmation of the integration site by Southern hybridization method or PCR method is necessary for all selected clones. Therefore, for example, if the herpes simplex virus-derived thymidine kinase (HSV-tk) gene that confers ganciclovir sensitivity is ligated outside the sequence homologous to the target site of the targeting vector for gene knockout, cells randomly inserted with the vector has the HSV-tk gene and cannot grow in a ganciclovir-containing medium, but cells targeted to the endogenous gene locus by homologous recombination are free of the HSV-tk gene and thus ganciclovir resistant, and are selected (negative selection). Alternatively, if, for example, a diphtheria toxin gene is ligated instead of the HSV-tk gene, cells randomly inserted with the vector are killed (positive selection) by the toxin produced by themselves, and homologous recombinants can also be selected in the absence of a drug.

For introduction of the targeting vector for gene knockout into the target cell, any of calcium phosphate coprecipitation method, electroporation method, lipofection method, retrovirus infection method, agglutination method, microinjection method, gene gun (particle gun) method, DEAE-dextran method, and the like can be used. As mentioned above, gene recombination in cells is mostly non-homologous, and the frequency of obtaining homologous recombinants is low. Therefore, the electroporation method is generally selected because it can easily process a large number of cells. For electroporation, the conditions generally used for gene transfer into animal cells can be used as they are. For example, the method can be performed by treating target cells in logarithmic growth phase with trypsin to disperse them into single cells, then suspending them in a medium at 10⁶ to 10⁸ cells/ml, transferring same into a cuvette, adding 10 to 100 µg of the targeting vector for gene knockout, and applying an electric pulse at 200 to 600 V/cm.

The target cell with the targeting vector for gene knockout integrated therein can also be verified by screening chromosomal DNAs isolated and extracted from colonies obtained by culturing single cells, by Southern hybridization or PCR method. When drug-resistant genes or reporter genes are used as other DNA fragments, transformants can be selected at the cell level by using the expression thereof as an index. For example, when a vector containing the nptII gene is used as a marker gene for positive selection, target cells after gene transfer treatment are cultured in a medium containing a neomycin antibiotic such as G418 and the like, and the emerged resistant colonies are selected as candidate transformants. When a vector containing the HSV-tk gene is used as a marker gene for negative selection, the cells are cultured in a medium containing ganciclovir, and the emerged resistant colonies are selected as candidate homologous recombinant cells. After transferring each of the obtained colonies to a culture plate and repeating trypsin treatment and medium exchange, some of the colonies are left for culture, and the rest are subjected to PCR or Southern hybridization to confirm the presence of the introduced DNA.

In addition, when a virus is used as a targeting vector for gene transfer, a method for infecting target cells with a virus containing a DNA containing an exogenous gene and a positive selection marker gene inserted between the 5'-arm and the 3'-arm and a negative selection marker gene outside the arm can be mentioned. The virus, the method for infecting cells, the method for selecting cells into which the vector has been integrated, and the like may be the same as the viruses and methods used for the targeting vector for gene knockout.

The target site of the targeting vector for gene transfer is not particularly limited as long as the exogenous gene can be expressed in the target cell. Examples of such site include safe harbor regions in the genome. Here, the safe harbor region is a site where the phenotype does not change even if an exogenous gene is integrated, and where the gene locus is open in many differentiated cells and the expression of the introduced factor is relatively stable, and is selected as a target site for integrating the exogenous gene into cells used as pharmaceuticals. Such safe harbor region includes AAVS1 (Adeno-associated virus integration site 1) region, CCR5 (C-C chemokine receptor 5) region, ROSA26 region, and the like. When an exogenous gene is introduced into a site other than the safe harbor region, an unexpected phenotype may be derived due to disruption of the gene at the introduced site, or the expression of the introduced exogenous gene may be suppressed. Therefore, the region must be carefully selected depending on the type of cells to be differentiated and used as a pharmaceutical. When an exogenous gene is introduced into the safe harbor region, the integration site of the exogenous gene is fixed, and it is expected that the difference in the expression level of the exogenous gene between the obtained homologous recombinants and the influence on other genes will be small.

Another embodiment for introducing an exogenous gene is the PiggyBac method. In the PiggyBac method, a transposon vector into which a DNA fragment containing an exogenous gene has been integrated and a transposase expression vector that expresses transposase are used. The genes and the like contained in the transposon vector and the transposase expression vector may be present separately in the above-mentioned separate vectors, or may be contained in a single vector. The transposon vector and the transposase expression vector may have, for example, the following constitutions.

In order to excise a DNA fragment containing an exogenous gene from the transposon vector by transposase, the transposon vector contains an inverted terminal repeat sequence (Inverted Terminal Repeat) at the 5'-upstream and 3'-downstream of the DNA fragment containing the exogenous gene. Transposase recognizes an inverted terminal repeat sequence contained in a transposon vector, and excises a DNA fragment containing an exogenous gene franked by the inverted terminal repeat sequences from the transposon vector.

In order to select target cells in which the exogenous gene has been integrated into the target site, the transposon vector preferably contains a drug resistance gene or a reporter gene in addition to the DNA fragment containing the exogenous gene. Here, the drug resistance gene and reporter gene may be the same as those used in the targeting vector for gene transfer.

The drug resistance and reporter genes are preferably under the control of any regulatory region for gene expression that can function in the target cell. The regulatory region for gene expression here may be the same as that used for the targeting vector for gene transfer.

The transposon vector preferably has a poly A signal at the downstream of the drug resistance or reporter gene, and the signal may be the same as that used for the targeting vector for gene transfer.

The transposase expression vector may contain drug resistance gene, reporter gene, regulatory region for gene expression, poly A signal, and the like in addition to a gene encoding transposase. The drug resistance gene, reporter gene, regulatory region for gene expression, and poly A signal may be the same as those contained in the transposon vector.

The same method as that used for the targeting vector for gene transfer may be used for the introduction of the transposon vector and the transposase expression vector into the target cell.

The cell in which the exogenous gene has been integrated into the target site may be selected by the same method as the method for selecting homologous recombinant cell into which the targeting vector for gene transfer has been integrated.

By means of the transposon vector, into which the DNA fragment of the exogenous gene is integrated, and the transposase expression vector that have been introduced as described above, the DNA fragment of the exogenous gene can be integrated into the transposase target sequence TTAA in the genome of the target cell. In this method, since the target sequence is TTAA unlike the homologous recombination method using the above-mentioned targeting vector for gene transfer, the site into which the exogenous gene is integrated cannot be limited. However, it is also possible to remove the exogenous gene integrated in the genome, without leaving a trace by expressing the transposase thereafter.

The modified pluripotent stem cell of the present invention may further express the following (c) and/or (d):
(c) an exogenous gene encoding interleukin 15 (IL-15)
(d) an exogenous gene encoding CD16.

IL-15 is produced by monocyte, macrophage, dendritic cell and the like, and causes proliferation and activation of cells that show tumoricidal action such as CTL and NK cell. Therefore, when IL-15 gene is introduced into and expressed in a pluripotent stem cell, NK cell per se differentiation-induced from the pluripotent stem cell as well as the surrounding T cells can be activated, thereby immune function of NK cell can be further enhanced.

The exogeneous IL-15 gene to be introduced is not particularly limited, for example, those in various forms described in WO 2020/045610 can be mentioned, and those encoding secretory IL-15 are preferable. The secretory IL-15 may be naturally occurring secretory IL-15 containing a native signal peptide, or for example, a modified IL-15 in which it is replaced with a heterologous signal peptide such as IL-2 signal peptide can also be preferably used.

In another preferable embodiment of the modified pluripotent stem cell of the present invention, an exogeneous gene encoding IL-15 receptor α (hereinafter also referred to as "IL-15Rα") is introduced and co-expressed with IL-15 gene. The gene may encode a full-length (membrane-bound) IL-15Rα or a soluble (secretory) IL-15Rα. Herein, the IL-15Rα gene may be introduced into the pluripotent stem cell as an expression construct separate to the IL-15 gene, or as an expression construct constructed so as to be expressed as a fused protein with IL-15. The description of WO 2020/045610 can be referred to as examples of such IL-15.

CD16 is a low-affinity receptor against Fc moiety of aggregated IgG. It can induce antibody-dependent cellular cytotoxicity against a target cell by binding to the Fc moiety of an antibody specific thereto. Therefore, when CD16 gene is introduced into and expressed in a pluripotent stem cell, NK cell differentiation-induced from the pluripotent stem cell has an enhanced antibody-dependent cellular cytotoxicity and can exert more potent tumoricidal activity.

CD16 has two kinds of isoforms, membrane-bound CD16a that is expressed in almost NK cells, a part of monocytes and the like, and GPI-anchored CD16b that is expressed in neutrophils. The CD16 used as an exogenous gene in the present invention may encode any of the isoforms as long as it can induce antibody-dependent cellular cytotoxicity, and CD16a is preferable. While the CD16 gene may be a wild-type gene, in a preferable embodiment, it may be a high-affinity variant with improved affinity with IgG Fc moiety, or a non-cleaved variant resistant to degradation by ADAM17. For example, as the high-affinity variant, F176V in which phenylalanine at position 176 is substituted with valine (F158V in which phenylalanine at position 158 of the mature type is substituted with valine) and the like can be mentioned, as non-cleaved variant, S197P in which serine at position 197 is substituted with proline and the like can be mentioned.

The modified pluripotent stem cell of the present invention may further express the following (e):
(e) an exogenous gene encoding NKG2D or NKG2D-CAR.

NKG2D is an activation receptor expressed in NK cell, transmits activation signal via stimulation by NKG2D ligand on a target cell, enhances cytotoxic activity of NK cell and enhances cytokine production such as IFN-γ and TNFα in NK cell. Therefore, when NKG2D gene is introduced into and expressed in a pluripotent stem cell, function of NK cell differentiation-induced from the pluripotent stem cell as an immunocompetent cell can be further enhanced.

As NKG2D gene to be introduced, an endogenous NKG2D gene in NK cell or a modified gene with enhanced function can be used. In addition to NKG2D gene, when an exogenous gene encoding a coupling factor DAP10 is co-expressed, the effect of NKG2D can be further increased. Alternatively, they can be introduced as a gene encoding a fused protein in which DAP10 is ligated with NKG2D intracellular domain.

Alternatively, NKG2D-CAR gene that encodes a fused protein in which NKG2D extracellular domain and the transmembrane domain, co-stimulating domain and signaling domain of a chimeric antigen receptor (CAR) can also be introduced. As the CAR used herein, combinations of transmembrane domains, co-stimulating domains and signaling domains used for conventional CAR-T cells can also be used, preferably, the transmembrane domain is from CD8a, the co-stimulating domain is from 2B4, and the signaling domain is from CD3z.

The above-mentioned exogenous genes encoding IL-15 (and IL-15Rα), CD16, and NKG2D can be constructed into an expression construct and introduced into a pluripotent stem cell in the same methods as the exogenous genes encoding CCR2B and CCL19.

### (II) Modified NK cell of the present invention

The thus-obtained modified pluripotent stem cell can be induced to differentiate into NK cell by a method known per se, for example, the method described in Matsubara et al. Biochem Biophys Res Commun. 2019 Jul 12; 515(1):1-8., and the like. Therefore, the present invention also provides NK cell and a progenitor cell thereof that are induced to differentiate from the modified pluripotent stem cell of the present invention (hereinafter also to be referred to as "the modified NK cell of the present invention", also including the progenitor cell).

Since the modified NK cell of the present invention highly expresses at least exogeneous CCR2B and CCL19, it can be efficiently homed to a target tissue expressing CCL2 (e.g., cancer tissue) and kill the target cell. In addition, it can exert a synergistic antitumor activity by migrating the surrounding T cells and dendritic cells expressing CCR7. In one embodiment, since it further expresses one or more exogenous gene(s) selected from IL-15 (and IL-15Rα), CD16, NKG2D (including NKG2D/DAP10 complex and NKG2D-CAR), additional function enhancement can be achieved by these function-enhancing factors. Therefore, the modified NK cell of the present invention can be, for example, used as a medicament for any disease against which a therapeutic effect can be exerted by killing cells expressing CCL2.

The medicament comprising the modified NK cell of the present invention as an active ingredient may be cultured using an appropriate medium prior to administering the NK cell to a subject. Also, the activation and/or proliferation of the NK cell can be maintained and amplified by adding a stimulating molecule to the medium. In addition, a serum or plasma may be added. Its amount to be added to the medium is not particularly limited, 0% by volume - 20% by volume can be exemplified, and the amount of serum or plasma to be used can be varied according to culture stage. For example, the serum or plasma can also be used while reducing its concentration stepwise. While the serum or plasma may be any of autologous or allogeneic, autologous one is preferable in view of safety.

The medicament comprising the modified NK cell of the present invention as an active ingredient is preferably used by parenteral administration to a subject. Examples of the parenteral administration method include intravenous, intraarterial, intramuscular, intraperitoneal, and subcutaneous administration methods and the like. The dosage is appropriately selected according to the condition, body weight, age, and the like of the subject. Generally, the number of cells per dose is 1×10⁶ to 1×10¹⁰, preferably 1×10⁷ to 1×10⁹, more preferably 5×10⁷ to 5×10⁸, in the case of a subject with body weight 60 kg. Moreover, it may be administered once or in multiple doses. The medicament can be in a known form suitable for parenteral administration, for example, injection or infusion.

The medicament may contain appropriately a pharmacologically acceptable excipient. The medicament may contain saline, phosphate buffered saline (PBS), medium and the like in order to maintain the cell stably. The medium includes, but are not limited to, media such as RPMI, AIM-V, and X-VIVO10. Pharmaceutically acceptable carriers (e.g., human serum albumin), preservative and the like can also be added to the medicament for the purpose of stabilization thereof.

The medicament comprising the modified NK cell of the present invention as an active ingredient can be a therapeutic drug for cancer. The cancer to which the medicament is applied is not particularly limited. For example, it includes, but are not limited to, acute lymphocytic cancer, alveolar rhabdomyosarcoma, bladder cancer, bone cancer, brain cancer (e.g., medulloblastoma), breast cancer, cancer of anus, anal canal or anus rectum, eye cancer, intrahepatic bile duct cancer, articular cancer, cervical, gall bladder or pleural cancer, cancer of nose, nasal cavity or middle ear, oral cancer, vulvar cancer, chronic myelogenous cancer, colorectal cancer, esophageal cancer, uterine cancer of the uterine cervix, fibrosarcoma, gastrointestinal carcinoid tumor, cancer of the head and neck (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myelogenous leukemia), liquid tumor, liver cancer, lung cancer (e.g., non-small cell lung cancer), lymphoma (e.g., Hodgkin lymphoma, non-Hodgkin's lymphoma, diffuse large B cell lymphoma, follicular lymphoma), malignant mesothelioma, mastocytoma, melanoma, multiple myeloma, nasopharynx cancer, ovarian cancer, pancreatic cancer; peritoneal, omental and mesentery cancer; pharyngeal cancer, prostate cancer, rectal cancer, skin cancer, small intestinal cancer, soft tissue cancer, solid tumor, gastric cancer, testicular tumor, thyroid cancer, ureteral cancer and the like.

### (III) CAR-NK cell

Meanwhile, an agent for cellular immunotherapy specific to a cell expressing an antigen can be provided by introducing the antigen-specific CAR into the modified NK cell of the present invention (CAR-NK cell).

The CAR-NK cell can be produced from the modified NK cell of the present invention using methods known per se as appropriately selected.

CAR is an artificially constructed hybrid protein containing the antigen-binding domain of an antibody (e.g., scFv) ligated with a T cell signaling domain. As a characteristic of CAR, an ability converting the specificity and reactivity of T cell to a selected target in non-MHC-restricted manner, using the antigen-binding property of a monoclonal antibody can be mentioned. The non-MHC-restricted antigen recognization confers antigen-recognizing ability to NK cell expressing CAR irrespective of antigen processing, thereby circumventing major mechanisms for tumor escape.

The CAR to be introduced into the modified NK cell of the present invention comprises the antigen-binding domain of an antibody capable of specifically recognizing a surface antigen to be recognized by the modified NK cell (e.g., cancer antigen peptide, surface receptor with increased expression in cancer cells etc.), an extracellular hinge domain, transmembrane domain and intracellular T cell signaling domain.

Examples of the surface antigen specifically recognized by the antigen-binding domain include, but are not limited to, surface receptors whose expression are increased in various cancer (e.g., acute lymphocytic cancer, alveolar rhabdomyosarcoma, bladder cancer, bone cancer, brain cancer (e.g., medulloblastoma), breast cancer, cancer of anus, anal canal or anus rectum, eye cancer, intrahepatic bile duct cancer, articular cancer, cervical, gall bladder or pleural cancer, cancer of nose, nasal cavity or middle ear, oral cancer, vulvar cancer, chronic myelogenous cancer, colorectal cancer, esophageal cancer, uterine cancer of the uterine cervix, fibrosarcoma, gastrointestinal carcinoid tumor, cancer of the head and neck (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myelogenous leukemia), liquid tumor, liver cancer, lung cancer (e.g., non-small cell lung cancer), lymphoma (e.g., Hodgkin lymphoma, non-Hodgkin's lymphoma, diffuse large B cell lymphoma, follicular lymphoma), malignant mesothelioma, mastocytoma, melanoma, multiple myeloma, nasopharynx cancer, ovarian cancer, pancreatic cancer; peritoneal, omental or mesentery cancer; pharyngeal cancer, prostate cancer, rectal cancer, skin cancer, small intestinal cancer, soft tissue cancer, solid tumor, gastric cancer, testicular tumor, thyroid cancer, ureteral cancer and the like), for example, CD19, EGF receptor, BCMA, CD30, Her2, ROR1, MUC16, CD20, mesothelin, B-cell mutation antigen, CD123, CD3, prostate-specific membrane antigen(PSMA), CD33, MUC-1, CD138, CD22, GD2, PD-L1, CEA, chondroitin sulfate proteoglycan-4, IL-13 receptor α chain, IgGκ light chain and the like, as well as cancer antigen peptide (e.g., peptide derived from WT1, GPC3, MART-1, gp100, NY-ESO-1, MAGE-A4 and the like) and the like.

The antigen binding domain used in the present invention is not particularly limited as long as it is an antibody fragment capable of specifically recognizing the target antigen. Considering ease in CAR production, a single chain antibody (scFv) in which light chain variable region and heavy chain variable region are ligated via a linker peptide is desirable. The arrangement of the light chain variable region and the heavy chain variable region in the single chain antibody is not particularly limited as long as the both can reconstitute a functional antigen-binding domain. In general, it can be designed in the order of light chain variable region-linker peptide-heavy chain variable region from its N-terminal side. As the linker peptide, linker peptides known per se as generally used in the production of single chain antibody can be used. A DNA encoding the light chain variable region and a DNA encoding the heavy chain variable region can be, for example, prepared by cloning a light chain gene and heavy chain gene, respectively, from antibody-producing cells and performing PCR using them as templates, and the like. Alternatively, they can be chemically synthesized using sequence information of existing antibody. Each DNA fragment obtained and a DNA encoding the linker peptide can be ligated by a suitable method to obtain a DNA encoding the single chain antibody. It is more preferable that a leader sequence is further added to the N-terminal side of the antigen-binding domain, in order to presenting the CAR on the surface of the modified NK cell.

As the extracellular hinge domain and transmembrane domain, T cell surface molecule-derived domains generally used in the art can be used as appropriate. For example, they include, but are not limited to, each domain derived from CD8α and CD28.

Examples of the intracellular signaling domain include, but are not limited to, one having CD3ζ chain, one further having a co-stimulating motif such as CD28, CD134, CD137, LCK, DAP10, ICOS, 4-1BB between the transmembrane domain and the CD3ζ chain, one having two or more co-stimulating motifs. Any domains generally used in the art can be used in combination.

Information on nucleic acid sequences encoding the extracellular hinge domain, transmembrane domain and intracellular signaling domain is well known in the art. One of ordinary skill in the art could easily obtain a DNA fragment encoding each domain from T cell based on such information.

Thus-obtained DNA fragments encoding the antigen-binding domain, extracellular hinge domain, transmembrane domain and intracellular signaling domain, respectively, can be ligated in a conventional method to obtain a DNA encoding the CAR.

The DNA encoding the CAR obtained can be inserted into an expression vector containing a regulatory region for gene expression functional in NK cell, preferably plasmid vector, as it is, or after adding a suitable linker and/or nuclear transition signal and the like. As the regulatory region for gene expression functional in NK cell, SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter and the like, which is constitutive in mammalian cells can be used, but are not limited thereto. Alternatively, gene promoters such as CD16, CD56 and NKG2D specifically expressed in NK cell can also be used.

### (IV) Production method for modified pluripotent stem cell and modified NK cell and reagent kit therefor

The present invention also provides a method of producing a genetically modified cell with enhanced homing function for cancer tissue and immunocompetent cell-recruiting function, comprising introducing the following (a) and (b):
(a) an exogenous gene encoding CCR2B
(b) an exogenous gene encoding CCL19
into a pluripotent stem cell.

In the above-mentioned production method, preparation of the exogeneous genes, gene transfer, selection of the genetically modified pluripotent stem cell and the like can be performed in the same manner as mentioned above. One or more exogenous gene(s) selected from IL-15 (and IL-15Rα), CD16, and NKG2D (including NKG2D/DAP10 complex and NKG2D-CAR) may also be further introduced.

The modified pluripotent stem cell obtained can be differentiation-induced into NK cell or progenitor cell thereof by a method known per se.

The present invention also provides a construct for producing a genetically modified cell, comprising the following (a) and (b):
(a) an exogenous gene encoding CCR2B
(b) an exogenous gene encoding CCL19.

Herein, the two exogeneous genes may be constructed so as to allow for polycistronic expression via 2A peptide or IRES, but can be preferably designed such that they are contained in separated constructs.

These constructs are located under the control of a functional regulatory region for gene expression (e.g., promoter, enhancer, polyadenylation signal etc.), and form expression cassettes. When the two exogeneous genes are contained in separate constructs, expression cassettes containing these constructs may be carried on a single vector, or carried on separate vectors.

In a preferable embodiment, the expression cassette is carried on a vector having a structure in which it is flanked by a pair of transposon inverted repeat sequences. The transposon vector can integrate an exogenous gene of interest onto the chromosome of the cell, by using in combination with a transposase expression vector, preferably PiggyBac transposase (PBase) expression vector. Therefore, the present invention also provides a kit for producing a genetically modified cell expressing the following (a) and (b):
(a) an exogenous gene encoding CCR2B
(b) an exogenous gene encoding CCL19,
comprising a transposon vector containing the above-mentioned expression cassette and a transposase expression vector.

The present invention is further specifically explained in the following by referring to Examples. However, they are mere examples and the present invention is not limited to these Examples.

### [Example]

### Production Example 1: Production of genetically modified iPS cells

As the parent strain of iPS cells, the iPS cell clone 06E (TC-1133HKK_06E_MCB) was used. This parent strain is hereinafter called as "unedited iPS cell". The following genes of interest were forcibly expressed in this unedited iPS cell. As a gene transfer method, PiggyBac method was used. For forced expression of cDNA of each factor, constitutively active EF1 alpha (EF1A) promoter region was used.

The following plasmid DNAs expressing respective genes of interest were prepared.
#CCR2B-expressing PiggyBac plasmid
#CCL19-expressing PiggyBac plasmid
#IL-15-expressing PiggyBac plasmid
#IL-15 receptor α-expressing PiggyBac plasmid
#secretory IL-15 receptor α-expressing PiggyBac plasmid #signal peptide-modified IL-15-expressing PiggyBac plasmid #CD16a-expressing PiggyBac plasmid
#NKG2D-expressing PiggyBac plasmid
#NKG2D-CAR-expressing PiggyBac plasmid
#DAP10-expressing PiggyBac plasmid

Also, the following plasmid DNA expressing a PiggyBac transposase was prepared.

### #hPBase-expressing plasmid

Each plasmid DNA used for gene transfer by PiggyBac method was constructed in the following method. A construct consisting of PiggyBac 3'-ITR sequence (SEQ ID NO: 1)-multicloning site (MCS) for restriction enzymes-PiggyBac 5'-ITR sequence (SEQ ID NO: 2) was artificially synthesized, and inserted into the restriction enzyme site of pHSG298 plasmid (Takara Bio Inc.). The plasmid DNA expressing each gene of interest was constructed by inserting EF1A promoter (amplified by PCR), each gene of interest (sequence thereof is described below), IRES (artificially synthesized: SEQ ID NO: 3), drug resistance gene (its sequence is described below) and human growth hormone poly A (artificially synthesized: SEQ ID NO: 4) into the MCS of the PiggyBac ITR plasmid produced. As a PCR template for PCR amplification of EF1A promoter, pBApo-EF1a Pur DNA plasmid (Takara Bio Inc.) was used.

As each gene of interest (artificially synthesized), any of CCR2B (artificially synthesized: SEQ ID NO: 5), CCL19 (artificially synthesized: SEQ ID NO: 6), IL-15 (artificially synthesized: SEQ ID NO: 7), full-length IL-15 receptor α (IL-15Rα) (artificially synthesized: SEQ ID NO: 8), soluble IL-15 receptor α (hereinafter also to be referred to as "sIL-15Rα") (artificially synthesized: SEQ ID NO: 9), signal peptide-modified IL-15 (hereinafter also to be referred to as "IL-2sp/IL-15") (artificially synthesized: SEQ ID NO: 10), CD16a (artificially synthesized: SEQ ID NO: 11), NKG2D (artificially synthesized: SEQ ID NO: 12), NKG2D-CAR (artificially synthesized: SEQ ID NO: 13) and DAP10 (artificially synthesized: SEQ ID NO: 14), or combination thereof was used.

As the drug resistance gene, any of Puromycin (artificially synthesized: SEQ ID NO: 15), Hygromycin (artificially synthesized: SEQ ID NO: 16), Zeocin (artificially synthesized: SEQ ID NO: 17) and Neomycin (artificially synthesized: SEQ ID NO: 18) was used.

The hPBase-expressing plasmid DNA was constructed in the following method. It was constructed by inserting EF1A promoter (amplified by PCR), PBase codon-optimized for human (artificially synthesized, SEQ ID NO: 19), and human growth hormone poly A (artificially synthesized: SEQ ID NO: 4) into a restriction enzyme site in the MCS of pHSG298 plasmid (Takara Bio Inc.).

The above-mentioned plasmid DNA expressing the factor to be introduced (one or plural according to purpose) and hPBase-expressing plasmid DNA were introduced into the unedited iPS cell 06E using electroporation method (Neon Transfection System, Thermo Fisher Scientific). From the day following plasmid DNA introduction, the cells were cultured in a liquid medium containing 1 to 4 kinds of drugs selected from puromycin, hygromycin, zeocin and neomycin for 3 to 7 days, and drug-resistant cells were selected. Thus, pools of iPS cells expressing introduced factor(s) were produced (Table 1).

**[Table 1]**

| cell line name | HGEC-0020 | HGEC-0024 | HGEC-0025 |
|---|---|---|---|
| Parent strain | Unedited iPS cell | HLA class I KO iPS cell | unedited iPS cell |
| introduced factor | CD16a | CD16a | CD16a |
| | CCR2B | CCR2B | CCR2B |
| | IL-15 | IL-15 | IL-2sp/IL-15 |
| | NKG2D | NKG2D | NKG2D |
| | DAP10 | DAP10 | DAP10 |
| | CCL19 | CCL19 | CCL19 |
| | Example 1 | Example 2 | Example 3 |

| Parent strain | unedited iPS cell | unedited iPS cell | unedited iPS cell |
|---|---|---|---|
| introduced factor | CCR2B | CCL19 | CD16a |
| | Example 4 | Example 5 | Example 6 |

| Parent | unedited iPS | unedited iPS | unedited iPS |
|---|---|---|---|
| strain | cell | cell | cell |
| introduced factor | IL-15 | NKG2D-CAR | NKG2D |
| | | | DAP10 |
| | Example 7 | Example 8 | Example 9 |

| Parent strain | unedited iPS cell | unedited iPS cell | unedited iPS cell |
|---|---|---|---|
| introduce factor | IL-2sp/IL-15 | IL-2sp/IL-15 | CCR2B |
| | IL-15Rα | sIL-15Rα | CCL19 |
| | Example 10 | Example 11 | Example 12 |

| Parent strain | unedited iPS cell |
|---|---|
| introduce factor | IL-2sp/IL-15 |
| | Example 13 |

Single cell cloning was performed from the above-mentioned iPS cell pools as necessary to isolate iPS clones expressing introduced factor(s).

### Production Example 2: Differentiation induction into NK cell

The iPS cell pools expressing gene(s) of interest or clones therefrom produced in Production Example 1 were expansion-cultured, and then differentiation-induced into NK cells, respectively. The differentiation induction of iPS cell into NK cell was performed according to the reference (Matsubara et al. Biochem Biophys Res Commun. 2019 Jul 12; 515(1):1-8.). As one of evaluation methods for differentiation potency into NK cell, cell surface expression of NK cell-specific proteins was determined by flow cytometry method. As NK cell markers, an anti-CD56 antibody (clone B159, BD Biosciences, Catalogue No. 555518), and anti-CD16 antibody (clone 3G8, BD Biosciences, Catalogue No. 555407) were used.

As a result, any of the iPS cells expressing the gene(s) of interest could be differentiated into NK cells.

### Experimental Example 1: Cell migration activity of CCR2B-expressing iNK cell

In iNK cells differentiation-induced form the CCR2B-expressing iPS cells (Example 4) obtained in Production Example 2, cell migration activity toward recombinant CCL2 protein-containing medium was examined using Boyden chamber.

Each of the iNK cells was labeled with CFSE (5- or 6-(N-Succinimidyloxycarbonyl)fluorescein 3',6'-diacetate), then 5×10⁵ cells were seeded into the upper chamber, the upper chamber was immersed in the lower chamber containing a medium containing 100 ng/mL of recombinant CCL2, and the cells were cultured at 37°C, in 5% CO₂ for 4 hr. Thereafter, the number of each of iNK cells migrated from the upper chamber to lower chamber was measured by a flow cytometer using CFSE fluorescence as an index. The results are shown in Fig. 1.

The cell number of the CCR2B-expressing iNK cells migrated to the lower chamber was remarkably greater than that of the control iNK cells, which were differentiation-induced from iPSCs into which an empty vector was introduced. These show that CCR2B-expressing iNK cell has a high migration activity for CCL2.

### Experimental Example 2: Cell migration activity of CCL19-expressing cell

Cell migration activity of peripheral blood mononuclear cells (PBMCs) toward the culture supernatant of iNK cells differentiation-induced from the CCL19-expressing iPS cells (Example 5) produced in Production Example 2 was examined using Boyden chamber.

The thawed and recovery-cultured PBMCs were labeled with CFSE, and then seeded into the upper chamber at 4×10⁵ cells/500 µL/well. They were immersed in the lower chamber containing the culture supernatant of the CCL19-expressing iNK cells, or the culture supernatant of the control iNK cells differentiation-induced from iPSCs into which an empty vector was introduced, and cultured at 37°C, in 5% CO₂ for 1.5 hr. Thereafter, the PBMCs migrated to the lower chamber were photographed by a fluorescence microscope using CFSE fluorescence as an index, and its cell number was measured using a flow cytometer. The results are shown in Fig. 2.

The culture supernatant of the CCL19-expressing iNK cells showed higher migration activity for the PBMCs as compared to the culture supernatant of the control iNK cells differentiation-induced from iPS cells into which an empty vector was introduced. These results show that CCL19-expressing iNK cell has an ability increasing the migration activity of PBMCs.

### Experimental Example 3: Cytotoxicity of CD16-expressing iNK cell

In the iNK cells (Example 6) differentiation-induced from the CD16-expressing iPS cells obtained in Production Example 2, it was confirmed by flow cytometry that CD16 was correctly expressed on the cell surface (Fig. 3, left).

Next, in the CD16-expressing iNK cells, the cytotoxic activity targeting A549 expressing EGFR was examined using LDH assay (Takara Bio LDH Cytotoxicity Detection Kit) in the presence of anti-EGFR antibody (Cetuximab). Specifically, adhered A549 cells were co-cultured with iNK cells in the presence of anti-EGFR antibody (Cetuximab) for 4 hr, and the amount of LDH released from A549 cells due to cytotoxicity by iNK cells was measured using the enzyme activity thereof as an index. The results are shown in Fig. 3, right. Compared with the control iNK cell differentiation-induced from empty vector-introduced iPS cells, about twice as high cytotoxic activity (ADCC activity) was confirmed in CD16-expressing iNK cells.

### Experimental Example 4: Cytokine secretion activity of IL-15-expressing iNK cell

iNK cells (Example 7) differentiation-induced from the IL-15-expressing iPS cells obtained in Production Example 2 were seeded at 2×10⁶ cells/mL, and after culturing for 4 days in the absence of IL-15, the number of the obtained viable cells was counted using a cell counting device, NucleoCounter NC-200. As a negative control, iNK cells differentiated from iPS cells introduced with an empty vector were used. The results are shown in Fig. 4.

Compared with the control iNK cells, more than twice the number of IL-15-expressing iNK cells was observed. Therefore, it was found that the proliferation and survival of iNK cells increased by expressing IL-15.

### Experimental Example 5: IFNγ secretion activity of NKG2D-expressing iNK cell

IFNγ production in various embodiments of NKG2D-expressing iNK cells obtained in Production Example 2 was examined. Respective NKG2D-iNK cells (NKG2D-CAR iNK cells (Example 8), NKG2D/DAP10-co-expressing iNK cells (Example 9)) were co-cultured for 24 hr with A549 cells expressing NKG2D ligands such as ULBP2, the culture supernatant was collected, and the expression level of IFNγ was confirmed by ELISA.

As a result of co-culture with A549 cells, a marked increase in the IFNγ production amount was observed in all NKG2D-iNK cells, compared with the control iNK cell differentiation-induced from iPS cells introduced with empty vector (Fig. 5). From the above results, it was found that NKG2D-specific IFNγ production was promoted in all NKG2D-expressing iNK cells.

### Experimental Example 6: Cell migration activity of CCR2B/CCL19-co-expressing iNK cell

In iNK cells (Example 12) differentiation-induced from the CCR2B/CCL19-co-expressing iPS cells obtained in Production Example 2, cell migration activity toward a medium containing recombinant CCL2 protein was examined using an Incucyte Clearview 96-well plate for chemotaxis (Sartorius). This plate is also divided into an upper chamber and a lower chamber, and cell migration can be evaluated using the same principle as in the case of the Boyden chamber.

After labeling each iNK cell with DiO (3,3'-Dioctadecyloxacarbocyanine perchlorate), 1×10⁴ cells were seeded in the upper chamber and immersed in the lower chamber containing a medium with or without 1 µg/mL recombinant CCL2. Thereafter, the cells were cultured at 37°C, in 5% CO₂, and the cells remaining in the upper chamber were quantified every 2 hr for 30 hr using DiO fluorescence as an index. The results thereof are shown in Fig. 6.

In CCR2B/CCL19-co-expressing iNK cells, a remarkable decrease in fluorescence in the upper chamber was observed when CCL2 was added to the lower chamber, compared to when CCL2 was not added. This result indicates that CCR2B/CCL19-co-expressing iNK cells migrated from the upper chamber to the lower chamber in a CCL2-dependent manner. On the other hand, in the control iNK cells differentiation-induced from empty vector-introduced iPS cells, a CCL2-dependent decrease in fluorescence was not observed in the upper chamber. From these, it was found that CCR2B/CCL19-co-expressing iNK cells exhibit high migration ability toward CCL2.

### Experimental Example 7: Cell migration activity of CCR2B/CCL19-co-expressing cell

The cell migration activity of dendritic cells (DCs) in the culture supernatant of the iNK cells (Example 12) differentiation-induced from the CCR2B/CCL19-co-expressing iPS cells obtained in Production Example 2 was examined using an Incucyte Clearview 96-well plate for chemotaxis (Sartorius).

DCs differentiation-induced from CD14 positive cells (monocytes) separated from peripheral blood mononuclear cells were labeled with DiO, and 8.4×10³ cells were seeded into the upper chamber. They were immersed, in the presence or absence of 10 ug/mL anti-CCL19 antibody (clone A15093C, BioLegend, Catalogue No.612803), in the lower chamber containing the culture supernatant of the CCR2B/CCL19-expressing iNK cells, or the culture supernatant of the control iNK cells differentiation-induced from iPSCs into which an empty vector was introduced. Thereafter, they were cultured at 37°C, 5% CO₂, and the DCs remaining in the upper chamber were quantified for 72 hr at 2 hr intervals using DiO fluorescence as an index. The results thereof are shown in Fig. 7.

When the culture supernatant of CCR2B/CCL19-co-expressing iNK cells was placed in the lower chamber, a decrease in fluorescence in the upper chamber was clearly observed compared to when the culture supernatant of control iNK cells was placed therein. This decrease in the fluorescence was suppressed by the addition of anti-CCL19 antibody. This indicates that DCs migrated to the culture supernatant of CCR2B/CCL19-co-expressing iNK cells in a CCL19-dependent manner. From the above results, it was found that CCR2B/CCL19-co-expressing iNK cells have high attracting activity against DC.

### Experimental Example 8: Effects of IL2sp/IL-15, full-length IL-15Rα and soluble IL-15Rα expression

iNK cells differentiation-induced from the IL2sp/IL-15-expressing iPS cells (Example 13), IL2sp/IL-15 and full-length IL15Rα-co-expressing iNK cells (Example 10), and IL2sp/IL-15 and soluble IL-15Rα-co-expressing iNK cells (Example 11) obtained in Production Example 2, and, as a control, iNK cells differentiation-induced from empty vector-introduced iPSC were cultured for 7 days in the absence of IL-15. On day 4 and day 7 of culture, the number of viable cells was counted using a cell counting device NucleoCounter NC-200. The results thereof are shown in Fig. 8. The measured values are expressed as % of the number of cells at the time of seeding.

Compared to control iNK cells, a very large number of viable cells were observed in IL2sp/IL-15-expressing iNK cells, IL2sp/IL-15 and full-length IL-15Rα-co-expressing iNK cells, and IL2sp/IL-15 and soluble IL-15Rα-co-expressing iNK cells. Compared to iNK expressing IL2sp/IL15 alone, more viable cells were observed in full-length IL15Rα- and soluble IL-15Rα-co-expressing iNK cells. The above results reveal that IL2sp/IL-15 expression enhances the proliferation and survival of iNK cells, and that this effect is further promoted by co-expressing full-length or soluble IL-15Rα.

### [Industrial Applicability]

The modified pluripotent stem cell and modified NK cell of the present invention are useful as active ingredients and starting materials for cellular immunotherapy, particularly cancer immunotherapy.

This application is based on a patent application No. 2021-184197 filed in Japan (filing date: November 11, 2021), the contents of which are incorporated in full herein.

## Claims

1. A pluripotent stem cell expressing the following (a) and (b):
(a) an exogenous gene encoding CC chemokine receptor 2 type B (CCR2B)
(b) an exogenous gene encoding CC chemokine ligand 19 (CCL19),
or an NK cell derived from the pluripotent stem cell, or a progenitor cell thereof.

2. The cell according to claim 1, further expressing the following (c) and/or (d):
(c) an exogenous gene encoding interleukin 15 (IL-15)
(d) an exogenous gene encoding CD16.

3. The cell according to claim 1 or 2, further expressing the following (e):
(e) an exogenous gene encoding NKG2D or NKG2D-CAR.

4. The cell according to claim 3, wherein (e) is NKG2D-CAR.

5. The cell according to claim 3, wherein (e) is NKG2D, further co-expressing:
(f) an exogenous gene encoding DAP10.

6. The cell according to any one of claims 2 to 5, expressing an exogenous gene encoding IL-15, wherein the IL-15 is a secretory IL-15.

7. The cell according to any one of claims 2 to 5, expressing an exogenous gene encoding IL-15, and co-expressing an exogenous gene encoding IL-15 receptor α (IL-15Rα).

8. The cell according to any one of claims 2 to 7, expressing an exogenous gene encoding CD16, wherein the CD16 is CD16 having a high affinity mutation or a non-cleavage mutation.

9. The cell according to claim 8, wherein the CD16 has a mutation selected from F176V (F158V) and S197P.

10. A medicament comprising the cell according to any one of claims 1 to 9.

11. The medicament according to claim 10, which is a therapeutic drug for cancer.

12. A method of producing a genetically modified cell with enhanced homing function for cancer tissue and immunocompetent cell-recruitment function, comprising introducing the following u (a) and (b) :
(a) an exogenous gene encoding CCR2,
(b) an exogenous gene encoding CCL19
into a pluripotent stem cell.

13. The method according to claim 12, further comprising inducing differentiation of the pluripotent stem cell, into which the exogeneous genes have been introduced, into NK cell or a progenitor cell thereof.

14. A construct for producing a genetically modified cell, comprising the following (a) and (b):
(a) an exogenous gene encoding CCR2B
(b) an exogenous gene encoding CCL19.

15. The construct according to claim 14, wherein the exogeneous genes are contained in separate constructs.

16. An expression cassette comprising a regulatory region for gene expression and the construct according to claim 14 or 15 under the control of the regulatory region.

17. A vector comprising the expression cassette according to claim 16.

18. The vector according to claim 17, having a structure in which the expression cassette is flanked by a pair of transposon inverted repeat sequences.

19. A kit for producing a genetically modified cell that expresses the following (a) and (b):
(a) an exogenous gene encoding CCR2B
(b) an exogenous gene encoding CCL19,
comprising the vector according to claim 18 and a transposase-expressing vector.

20. The kit according to claim 19, wherein the transposase is PiggyBac transposase.
